# FASCICULE DE BREVET EUROPEEN

(11) **EP 3 745 948 B1**
(45) Date de publication et mention de la délivrance du brevet: **05.03.2025**
(21) Numéro de dépôt: 19707856.1
(22) Date de dépôt: 28.01.2019
(51) Int. Cl.: A61B 5/00, A61B 5/024, A61B 5/145, A61B 5/0205

(54) **DISPOSITIF DE TÉLÉSURVEILLANCE D'UN PATIENT SOUFFRANT D'INSUFFISANCE CARDIAQUE CHRONIQUE, SYSTÈME ET PROGRAMME ASSOCIES**
VORRICHTUNG ZUR ENTFERNTEN ÜBERWACHUNG EINES PATIENTEN MIT CHRONISCHEM HERZVERSAGEN UND ZUGEHÖRIGES SYSTEM UND PROGRAMM
DEVICE FOR REMOTELY MONITORING A PATIENT WITH CHRONIC HEART FAILURE, AND ASSOCIATED SYSTEM AND PROGRAM

(30) Priorité: 30.01.2018 FR 1850760
(43) Date de publication de la demande: 09.12.2020
(73) Titulaire: Edevice, 33700 Merignac (FR)
(72) Inventeur: RAPHAEL, Pierre, 37000 TOURS (FR); JOURDAIN, Patrick, 95300 PONTOISE (FR)
(74) Mandataire: Novagraaf Technologies
(86) Numéro de dépôt international: PCT/FR2019/050173
(87) Numéro de publication internationale: WO 2019/150023

(56) Documents cités:
- US-A1- 2015 342 540
- US-A1- 2017 100 079
- DANIEL BENATAR ET AL: "Outcomes of Chronic Heart Failure", ARCHIVES OF INTERNAL MEDICINE., vol. 163, no. 3, 10 February 2003 (2003-02-10), US, pages 347, XP055511087, ISSN: 0003-9926, DOI: 10.1001/archinte.163.3.347
- PATRIK LYNGÅ ET AL: "Weight monitoring in patients with severe heart failure (WISH). A randomized controlled trial", EUROPEAN JOURNAL OF HEART FAILURE, vol. 14, no. 4, 1 April 2012 (2012-04-01), NL, pages 438 - 444, XP055510949, ISSN: 1388-9842, DOI: 10.1093/eurjhf/hfs023
- MOTONORI HAYASHI ET AL: "Nocturnal Oxygen Desaturation as a Predictive Risk Factor for Coronary Restenosis After Coronary Intervention", CIRCULATION JOURNAL, vol. 69, no. 11, 1 November 2005 (2005-11-01), JP, pages 1320 - 1326, XP055511037, ISSN: 1346-9843, DOI: 10.1253/circj.69.1320

## Description

### Domaine technique

La présente invention se rapporte à un dispositif de télésurveillance d'un patient souffrant d'insuffisance cardiaque chronique, ainsi qu'à un système de télésurveillance comprenant un tel dispositif et un programme d'ordinateur utilisant un tel dispositif.

### Etat de la technique

L'insuffisance cardiaque chronique est une maladie qui a plusieurs spécificités. Elle est à la fois commune et grave. L'insuffisance cardiaque chronique affecte environ 1% de la population dans les pays développés. Cela inclut presque 800 000 patients en France. C'est une maladie qui progresse particulièrement à cause du vieillissement inexorable de la population avec un nombre d'hospitalisations qui augmente régulièrement (14% d'augmentation en France entre 2002 et 2008). Cette tendance est confirmée dans d'autres pays comme les USA. C'est une maladie qui est également associée à une forte mortalité et également à un fort taux de réhospitalisation. Cela est en partie dû à la sévérité « per se » de la maladie mais aussi à une gestion non-optimale de la maladie ainsi qu'à une éducation des patients et des soignants mal formalisée. Ainsi, la majorité des patients hospitalisés ont montré des signes cliniques de décompensation et ce, jusqu'à 5 jours avant l'hospitalisation. Une bonne gestion de ces signes aurait pu éviter aux patients de se rendre aux urgences et une hospitalisation en urgence, grâce à une prise en charge et des soins en ambulatoire.

C'est sur ce constat qu'apparait l'intérêt d'utiliser la télémédecine pour l'insuffisance cardiaque chronique. La télémédecine est la surveillance de données cliniques ou biologiques au domicile du patient avec le transfert de ces données à distance soit vers une structure spécialisée souvent gérée par des infirmières, soit vers le médecin généraliste traitant ou le cardiologue.

Beaucoup de types de données ont été étudiés et suivis dans la littérature scientifique et médicale pour suivre l'état de santé. On peut citer des données se rapportant à l'état de santé du patient, des données cliniques comme, par exemple, le poids et ses variations, la pression artérielle, la fréquence cardiaque, l'activité physique, etc. ou des données biologiques comme par la mesure par impédance , par la mesure directe de la pression intracardiaque, ou par l'électrocardiographie, etc.. Malheureusement les résultats sont extrêmement variables en fonction des études et de ce fait, à ce jour, l'utilisation de la télémédecine n'est pas validée et recommandée dans la gestion de l'insuffisance cardiaque chronique.

De plus l'utilisation de la télémédecine a montré de nombreuses limitations soulignées par de nombreuses études. En particulier, la faible sensibilité et la spécificité des signes cliniques génèrent beaucoup d'alertes ce qui en rend la gestion par le patient et le médecin difficile. La télémédecine est également coûteuse avec une méthodologie complexe. De plus elle pose des problèmes médico-légaux relatifs à la responsabilité de ceux qui interprètent les données à distance de télémédecine.

Il existe donc un réel besoin pour un dispositif de télésurveillance de patients atteints d'insuffisance cardiaque chronique palliant ces défauts, inconvénients et obstacles de l'art antérieur, en particulier d'un dispositif relativement peu coûteux et suffisamment fiable dans ses alertes pour minimiser les fausses alarmes.

Le document « Outcomes of Chronic heart Failure » de Daniel BENATAR et al, 10 février 2003 (Archives of Internal Medecine, XP055511087) décrit un système de télémédecine comportant un appareillage permettant de suivre le poids, la pression sanguine, la fréquence cardiaque et le taux de saturation en oxygène. Dans cet appareillage une analyse de détection d'un franchissement de seuil est effectuée paramètre par paramètre.

D'autres documents décrivent des dispositifs et/ou méthodes pour le suivi de patients atteints d'insuffisance cardiaque chronique. On peut citer par exemple les documents suivants:
- le document "Weight monitoring in patients with severe heart failure (WISH). A randomized controlled trial", de Patrick Lyngå et al, European Journal of Heart Failure", 1 avril 2012, XP0555510949;
- le document "Nocturnal Oxygen Desaturation as a Predictive Risk Factor for Coronary Restenosis After Coronary Intervention" par Motonori Hayashi et al, Circulation Journal, 1 novembre 2005, XP055511037;
- les document brevets publiés respectivement sous les numéros US 2015/342540 A1 (An Qi et al) et US 2017/100079 A1 (Cuba Gyllenstein Illapha Gustav Lars et al).

### Résumé de l'invention

Pour résoudre un ou plusieurs des inconvénients cités précédemment, l'invention concerne selon un premier aspect un dispositif de télésurveillance d'un patient souffrant d'insuffisance cardiaque chronique suivant la revendication 1.

Un tel dispositif a l'avantage d'utiliser des moyens de mesure, balance et oxymètre, d'un coût relativement faible et de fiabiliser le déclenchement de l'alerte par la combinaison de trois types de mesure différents. L'algorithme est simple et générique. En outre, l'utilisation de seuils différents pour ces trois types de mesure selon que l'on procède à la vérification des premières conditions ou des deuxièmes condistions permet de fiabiliser le déclenchement en ce que des déclenchements intempestifs peuvent être évités tout en garantissant que les alertes déclenchées sont pertinentes.

La présente description décrit des caractéristiques ou des modes de réalisation particuliers, utilisables seuls ou en combinaison, selon lesquels :
- la première augmentation limite de poids est égale à 4kg ; et
- la seconde augmentation limite de poids est égale à 2kg ;
- la période prédéterminée est de 4 jours ; et
- le premier taux de saturation en oxygène dans le sang est égal à 90% ; et
- le second taux de saturation en oxygène dans le sang est égal à 95% ; et
- la première fréquence cardiaque est égale à 110 pulsations/mn ; et
- la seconde fréquence cardiaque est égale à 90 pulsations/mn ;
- le dispositif de télésurveillance comprend une horloge associée à une interface homme-machine pour rappeler au patient de prendre ses mesures quotidiennes dans une plage horaire prédéterminée ; et/ou
- le dispositif de télésurveillance comprend une quatrième interface de communication adaptée pour transmettre les mesures sur un serveur distant.

L'invention concerne selon un deuxième aspect un système de télésurveillance d'un patient souffrant d'insuffisance cardiaque suivant la revendication 6.

L'invention concerne selon un troisième aspect un produit programme d'ordinateur suivant la revendication 7.

### Brève description des figures

L'invention sera mieux comprise à la lecture de la description qui suit, faite uniquement à titre d'exemple, et en référence aux figures en annexe dans lesquelles :
- La figure 1 représente un système de télésurveillance selon un mode de réalisation de l'invention ;
- La figure 2 représente une architecture d'un dispositif de télésurveillance, élément du système de la figure 1 ; et
- La figure 3 représente un ordinogramme d'un procédé mis en œuvre par le système de la figure 1.

Les procédés présentés ne font pas partie de l'invention revendiquée.

### Modes de réalisation

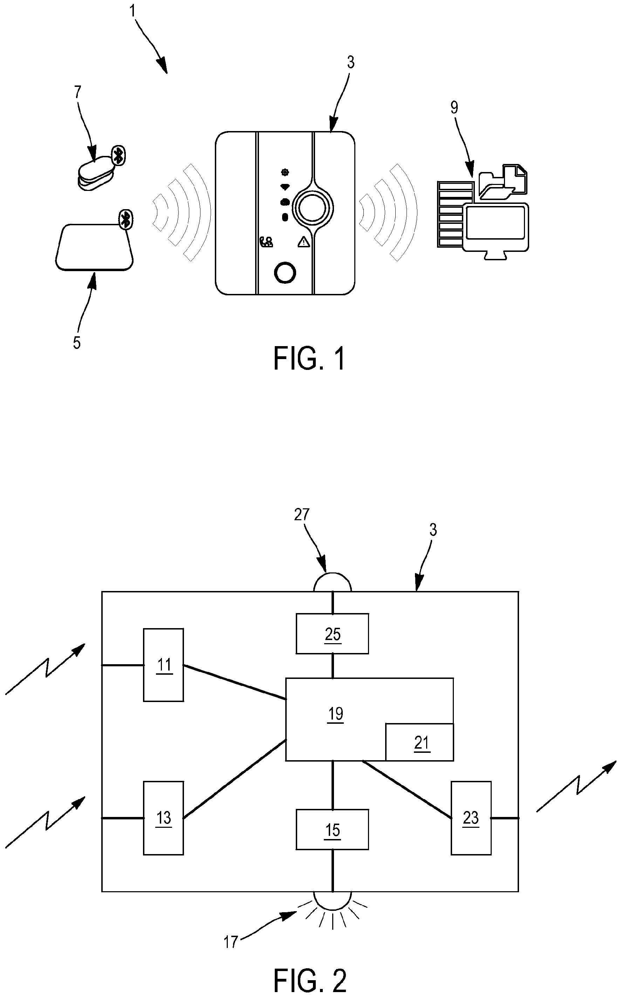

En référence à la figure 1, un système de télésurveillance 1 d'un patient souffrant d'insuffisance cardiaque chronique comprend un dispositif de télésurveillance 3 connecté d'une part à une balance 5 et un oxymètre 7 et d'autre part à un serveur 9 de gestion de soin installé à distance.

Le dispositif de télésurveillance 3, la balance 5 et l'oxymètre 7 sont installés au domicile du patient alors que le serveur 9 de gestion de soin est installé chez un prestataire assurant la gestion du système.

La connexion du dispositif de télésurveillance 3 avec la balance 5 et l'oxymètre 7 est établie de préférence par une liaison sans-fil courte portée de type Bluetooth.

La connexion du dispositif de télésurveillance 3 avec le serveur 9 de gestion de soin est assurée par une liaison WiFi avec un modem-routeur (non représenté) qui communique avec le réseau internet.

De façon plus détaillée, le dispositif de télésurveillance 3 comprend, figure 2, une première interface de communication 11 adaptée pour communiquer avec la balance 5 afin recueillir une mesure du poids du patient.

Une deuxième interface de communication 13 est adaptée pour communiquer avec l'oxymètre afin de recueillir des mesures de la fréquence cardiaque et du taux de saturation en oxygène dans le sang du patient.

Le dispositif de télésurveillance 3 comprend également une troisième interface de communication 15 adaptée pour transmettre une alerte au patient. Par exemple, comme illustré sur la figure 2, l'alerte est transmise par un voyant lumineux 17 qui est éteint en mode normal et qui clignote lors d'une alerte.

Un calculateur 19 est connecté aux trois interfaces 11, 13, 15. Il comprend une mémoire de stockage 21 adaptée pour stocker les mesures recueillies, le calculateur étant adapté pour générer une alerte lorsque les mesures recueillies indiquent une décompensation cardiaque débutante probable chez le patient.

Le calculateur 19 est également connecté à une quatrième interface de communication 23 adaptée pour transmettre les mesures recueillies au serveur 9.

Le dispositif de télésurveillance 3 comprend également une horloge 25 associée à une interface homme-machine 27 pour rappeler au patient de prendre ses mesures dans une plage horaire prédéterminée.

Comme pour l'alerte, cette interface homme-machine 27 est par exemple un affichage lumineux.

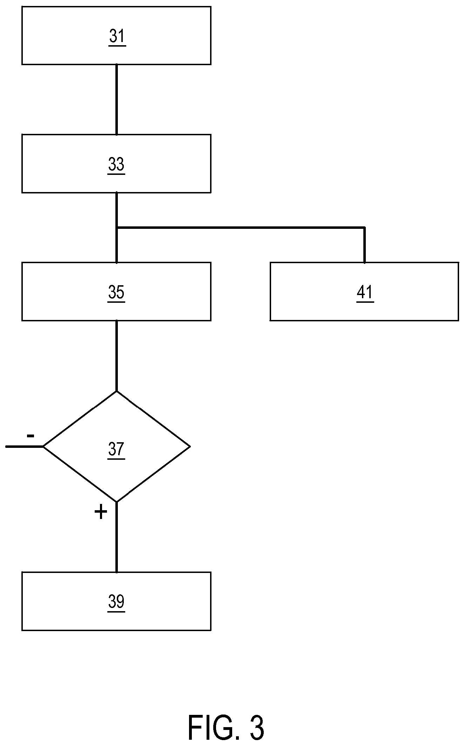

Le fonctionnement du système de télésurveillance 1 et, plus particulièrement, celui du dispositif de télésurveillance 3, vont maintenant être décrits en relation avec la figure 3.

En préliminaire, des seuils d'alerte sur les différentes mesures, poids, fréquence cardiaque et taux de saturation en oxygène dans le sang, sont prédéfinis, étape 31.

Concernant le poids, on définit une première augmentation limite de poids et une seconde augmentation limite de poids strictement inférieure à la première augmentation limite de poids.

Par exemple, on va définir que la première augmentation limite de poids est 4 kg et la seconde augmentation limite de poids est 2 kg.

Une augmentation rapide du poids du patient au-dessus de ces deux valeurs limites peut être le signe d'un épisode de rétention d'eau.

De même, on définit un premier taux de saturation en oxygène dans le sang et un second taux de saturation en oxygène dans le sang strictement supérieur au premier taux de saturation en oxygène dans le sang.

Par exemple, le premier taux de saturation en oxygène dans le sang est égal à 90% et le second taux de saturation en oxygène dans le sang est égal à 95%.

Un taux de saturation en oxygène dans le sang faible peut être le premier signe d'un œdème aigu du poumon.

Enfin, on définit une première fréquence cardiaque et une seconde fréquence cardiaque strictement inférieure à la première fréquence cardiaque.

Par exemple, la première fréquence cardiaque est égale à 110 pulsations/mn et la seconde fréquence cardiaque est égale à 90 pulsations/mn.

Après installation des différents éléments du système de télésurveillance 1 chez le patient et la vérification du bon fonctionnement et que les différentes connexions se réalisent sans difficulté, le protocole de surveillance est mis en place.

Tous les jours, le patient mesure, étape 33, son poids, sa fréquence cardiaque et son taux de saturation en oxygène dans le sang.

De façon à ce que les mesures soient comparables, la séance de mesure doit se faire toujours dans les mêmes conditions, de préférence quotidiennement. Ainsi, il est préférable que celle-ci ait lieu au lever, à jeun et après être passé aux toilettes.

Cela définit donc une plage horaire pour la prise des mesures. Pour garantir l'effectivité de l'opération, le dispositif de télésurveillance 3 utilise son horloge 25 associée à l'interface homme-machine 27 pour rappeler au patient de prendre les mesures.

Par ailleurs, la première mesure de poids, ou la première mesure après une période sans mesure de plus de 4 jours ou une première mesure obtenue après une remise à zéro du dispositif de surveillance et/ou de son calculateur, peut être utilisée pour définir le poids nominal du patient.

Une fois les mesures faites et acquises par le dispositif de télésurveillance 3, le calculateur 19 va les analyser, étape 35, selon les règles de décision suivantes.

Il est probable qu'il y a une décompensation cardiaque débutante si une des premières conditions suivantes est remplie :
- le poids a augmenté d'une valeur strictement supérieure à la première augmentation limite de poids par rapport au poids nominal du patient ;
- le poids a augmenté, entre deux mesures prises sur une période prédéterminée strictement supérieure à 2 jours (cette première période est par exemple égale à 4 jours), d'une valeur strictement supérieure à la première augmentation limite de poids ;
- le taux de saturation en oxygène dans le sang est strictement inférieur au premier taux de saturation en oxygène dans le sang;
- la fréquence cardiaque est strictement supérieure à la première fréquence cardiaque; ou,

Il est probable qu'il y a une décompensation cardiaque débutante si deux des deuxièmes conditions suivantes sont remplies simultanément, alors qu'aucune des premières conditions précédentes n'est remplie :
- le poids a augmenté entre deux mesures, prises sur une deuxième période égale à 2 jours consécutifs, d'une valeur strictement supérieure à la seconde augmentation limite de poids ;
- le taux de saturation en oxygène dans le sang est strictement inférieur au second taux de saturation en oxygène dans le sang ;
- la fréquence cardiaque est strictement supérieure à la seconde fréquence cardiaque.

En utilisant les données numériques ci-dessus, et en considérant que la période prédéterminée indiquée ci-dessus est de 4 jours, ces règles se traduisent en :
- le poids a augmenté de plus de 4kg par rapport au poids nominal du patient ;
- le poids a augmenté de plus de 4kg en 4 jours ;
- le taux de saturation en oxygène dans le sang est strictement inférieur à 90% ;
- la fréquence cardiaque est strictement supérieure à 110 pulsations/mn ; ou
- deux des conditions suivantes sont remplies simultanément :
   - le poids a augmenté de plus de 2kg en 2 jours ;
   - le taux de saturation en oxygène dans le sang est strictement inférieur à 95% ;
   - la fréquence cardiaque est strictement supérieure à 90 pulsations/mn.

Si, selon les règles ci-dessus, le calculateur 19, considère, étape 37, qu'il est probable qu'une insuffisance cardiaque débute, alors il déclenche, étape 39, une alerte pour le patient.

En parallèle à cette analyse, les mesures sont transmises, étape 41, au serveur 9.

Il est noté que les périodes utilisées pour la vérification des premières conditions et deuxièmes conditions peuvent être adaptées. La première période (4 jours dans les exemples précédents) utilisée pour la vérification des premières conditions est supérieure à la deuxième période (2 jours dans les exemples précédents) utilisée pour la vérification des deuxièmes conditions.

Recevant l'alerte, le patient peut alors prendre rendez-vous chez son médecin, ou l'appeler. Celui-ci a accès aux mesures stockées sur le serveur 9 et est alors à même de prendre une décision de soin : hospitalisation, consultation plus complète, etc. en fonction du diagnostic qu'il peut poser.

Il est important de noter que le système n'est pas destiné à remplacer la prise en charge médicale habituelle du patient mais à alerter le patient pour que celui-ci ait une démarche active de prise en charge de son état de santé.

L'invention a été illustrée et décrite en détail dans les dessins et la description précédente. Celle-ci doit être considérée comme illustrative et donnée à titre d'exemple et non comme limitant l'invention a cette seule description. De nombreuses variantes de réalisation sont possibles dans le cadre de l'invention définie par les revendications.

Par exemple, la connexion entre le dispositif de télésurveillance 3 et la balance 5 ou l'oxymètre 7 peut être assurée par une liaison sans-fil de type WiFi ou bien par une liaison électrique filaire telle qu'une liaison série. Bien que moins pratique, il est également envisageable de faire rentrer les données manuellement par le patient.

De même, la connexion entre le dispositif de télésurveillance 3 et le serveur 9 de gestion de soin peut être une liaison filaire de type Ethernet ou bien une liaison sans-fil via un réseau téléphonique selon une norme de type 3G ou 4G. En fonction de l'environnement de fonctionnement, l'homme du métier saura choisir le mode de connexion le plus approprié pour transférer les données de façon fiable et à moindre coût.

De même, le patient peut être alerté par le dispositif de télésurveillance par différents moyens sonores, tactiles ou visuels. Par exemple, l'alerte peut être transmise à une montre connectée portée par le patient et qui va générer une vibration à son poignet.

Dans les revendications, le mot « comprenant » n'exclue pas d'autres éléments et l'article indéfini « un/une » n'exclue pas une pluralité.

## Revendications

1. Dispositif de télésurveillance (3) d'un patient souffrant d'insuffisance cardiaque chronique, ledit dispositif comprenant :
• une première interface de communication (11) adaptée pour communiquer avec une balance (5) afin de recueillir une mesure du poids du patient ;
• une deuxième interface de communication (13) adaptée pour communiquer avec un oxymètre (7) afin de recueillir des mesures de la fréquence cardiaque et du taux de saturation en oxygène dans le sang du patient ;
• une troisième interface de communication (15) adaptée pour transmettre une alerte au patient ; et
• un calculateur (19) connecté aux trois interfaces (11, 13, 15), comprenant une mémoire de stockage (21) adaptée pour stocker les mesures recueillies, le calculateur étant adapté pour déclencher une alerte lorsque les mesures recueillies indiquent une décompensation cardiaque débutante probable chez le patient ;
le calculateur étant configuré pour :
- obtenir un poids nominal du patient et des valeurs prédéterminées comprenant :
• une première augmentation limite de poids ;
• une seconde augmentation limite de poids strictement inférieure à la première augmentation limite de poids ;
• un premier taux de saturation en oxygène dans le sang ;
• un second taux de saturation en oxygène dans le sang strictement supérieur au premier taux de saturation en oxygène dans le sang ;
• une première fréquence cardiaque ; et
• une seconde fréquence cardiaque strictement inférieure à la première fréquence cardiaque,
- déterminer à partir des mesures recueillies si l'une quelconque des premières conditions suivantes est remplie :
• le poids a augmenté plus que la première augmentation limite de poids par rapport au poids nominal du patient ;
• le poids a augmenté, entre deux mesures prises sur une période prédéterminée strictement supérieure à 2 jours, d'une valeur strictement supérieure à la première augmentation limite de poids ;
• le taux de saturation en oxygène dans le sang est strictement inférieur au premier taux de saturation en oxygène dans le sang;
• la fréquence cardiaque est strictement supérieure à la première fréquence cardiaque;
- déterminer qu'une décompensation cardiaque débutante est probable lorsqu'au moins une des premières conditions est remplie ;
- déterminer à partir des mesures recueillies si l'une quelconque des deuxièmes conditions suivantes est remplie :
• le poids a augmenté entre deux mesures prises sur 2 jours consécutifs, d'une valeur strictement supérieure à la seconde augmentation limite de poids ;
• le taux de saturation en oxygène dans le sang est strictement inférieur au second taux de saturation en oxygène dans le sang ;
• la fréquence cardiaque est strictement supérieure à la seconde fréquence cardiaque.
- déterminer qu'une décompensation cardiaque débutante est probable lorsque deux des deuxièmes conditions sont remplies simultanément, alors qu'aucune des premières conditions n'est remplie.

2. Dispositif selon la revendication 1, dans lequel le poids nominal correspond à une première mesure de poids ou une première mesure après une remise à zéro du dispositif ou une première mesure après une période sans mesure de plus de 4 jours.

3. Dispositif selon la revendication 1 ou 2, **caractérisé en ce que**
• la première augmentation limite de poids est égale à 4kg ;
• la seconde augmentation limite de poids est égale à 2kg ;
• la période prédéterminée est de 4 jours ;
• le premier taux de saturation en oxygène dans le sang est égal à 90% ;
• le second taux de saturation en oxygène dans le sang est égal à 95% ;
• la première fréquence cardiaque est égale à 110 pulsations/mn ;
• la seconde fréquence cardiaque est égale à 90 pulsations/mn.

4. Dispositif selon la revendication 1, 2 ou 3, **caractérisé en ce qu'**il comprend une horloge (25) associée à une interface homme-machine (27) pour rappeler au patient de prendre ses mesures chaque jour dans une plage horaire prédéterminée.

5. Dispositif selon l'une quelconque des revendications précédentes, **caractérisé en ce qu'**il comprend une quatrième interface de communication (23) adaptée pour transmettre les mesures sur un serveur distant (9).

6. Système de télésurveillance d'un patient souffrant d'insuffisance cardiaque comprenant :
• un dispositif de télésurveillance (3) selon l'une quelconque des revendications 1 à 5 ;
• une balance (5) ;
• un oxymètre (7) ; la balance (5) et l'oxymètre (7) étant connectés au dispositif de télésurveillance (3) ; et
• un serveur distant (9) connecté au dispositif de télésurveillance (3) et adapté pour recueillir, stocker et visualiser l'historique des mesures du patient.

7. Produit programme d'ordinateur **caractérisé en ce qu'**il comprend des instructions de code de programme pour la mise en œuvre par un dispositif de télésurveillance d'un procédé de télésurveillance d'un patient souffrant d'insuffisance cardiaque, le procédé comprenant :
- une obtention, via une première interface de communication (11) du dispositif de télésurveillance avec une balance (5), de mesures du poids du patient,
- une obtention, via une deuxième interface de communication (13) du dispositif de télésurveillance avec un oxymètre (7), de mesures de la fréquence cardiaque et du taux de saturation en oxygène dans le sang du patient ;
- une obtention de valeurs prédéterminées comprenant :
• une première augmentation limite de poids ;
• une seconde augmentation limite de poids strictement inférieure à la première augmentation limite de poids ;
• un premier taux de saturation en oxygène dans le sang ;
• un second taux de saturation en oxygène dans le sang strictement supérieur au premier taux de saturation en oxygène dans le sang ;
• une première fréquence cardiaque ; et
• une seconde fréquence cardiaque strictement inférieure à la première fréquence cardiaque,
- une détermination à partir des mesures de poids, de fréquence cardiaque et de taux de saturation en oxygène obtenues pour le patient si l'une quelconque des premières conditions suivantes est remplie :
• le poids a augmenté d'une valeur strictement supérieure à la première augmentation limite de poids par rapport au poids nominal du patient ;
• le poids a augmenté, entre deux mesures prises sur une période prédéterminée strictement supérieure à 2 jours, d'une valeur strictement supérieure à la première augmentation limite de poids ;
• le taux de saturation en oxygène dans le sang est strictement inférieur au premier taux de saturation en oxygène dans le sang;
• la fréquence cardiaque est strictement supérieure à la première fréquence cardiaque;
- un déclenchement d'une alerte signalant qu'une décompensation cardiaque débutante est probable lorsqu'au moins une des premières conditions est remplie ;
- une détermination à partir des mesures obtenues si l'une quelconque des deuxièmes conditions suivantes est remplie :
• le poids a augmenté entre deux mesures prises sur 2 jours consécutifs, d'une valeur strictement supérieure à la seconde augmentation limite de poids ;
• le taux de saturation en oxygène dans le sang est strictement inférieur au second taux de saturation en oxygène dans le sang ;
• la fréquence cardiaque est strictement supérieure à la seconde fréquence cardiaque ;
- un déclenchement d'une alerte signalant qu'une décompensation cardiaque débutante est probable lorsque deux des deuxièmes conditions sont remplies simultanément, alors qu'aucune des premières conditions n'est remplie.

8. Produit programme d'ordinateur selon la revendication 7, dans lequel le poids nominal correspond à une première mesure de poids, une première mesure après une remise à zéro ou une première mesure après une période sans mesure de plus de 4 jours.

9. Produit programme d'ordinateur selon la revendication 7 ou 8, **caractérisé en ce que**
• la première augmentation limite de poids est égale à 4kg ;
• la seconde augmentation limite de poids est égale à 2kg ;
• la période prédéterminée est de 4 jours ;
• le premier taux de saturation en oxygène dans le sang est égal à 90% ;
• le second taux de saturation en oxygène dans le sang est égal à 95% ;
• la première fréquence cardiaque est égale à 110 pulsations/mn ;
• la seconde fréquence cardiaque est égale à 90 pulsations/mn.

## Patentansprüche

1. Vorrichtung (3) für eine Fernüberwachung eines Patienten, der an chronischer Herzinsuffizienz leidet, wobei die Vorrichtung umfasst:
• eine erste Kommunikationsschnittstelle (11), die angepasst ist, um mit einer Waage (5) zu kommunizieren, um eine Messung des Gewichts des Patienten zu erfassen;
• eine zweite Kommunikationsschnittstelle (13), die angepasst ist, um mit einem Oximeter (7) zu kommunizieren, um Messungen der Herzfrequenz und des Sauerstoffsättigungsgrads in dem Blut des Patienten zu erfassen;
• eine dritte Kommunikationsschnittstelle (15), die angepasst ist, um eine Warnung an den Patienten zu übertragen; und
• einen Rechner (19), der mit den drei Schnittstellen (11, 13, 15) verbunden ist, umfassend einen Speicher (21), der angepasst ist, um die erfassten Messungen zu speichern, wobei der Rechner angepasst ist, um einen Alarm auszulösen, wenn die erfassten Messungen eine wahrscheinliche beginnende Herzdekompensation bei dem Patienten anzeigen;
wobei der Rechner konfiguriert ist zum:
- Erhalten eines Sollgewichts des Patienten und vorbestimmter Werte, umfassend:
• eine erste Erhöhunggrenze des Gewichts;
• eine zweite Erhöhunggrenze des Gewichts, die streng niedriger als die erste Erhöhunggrenze des Gewichts ist;
• einen ersten Sauerstoffsättigungsgrad in dem Blut;
• einen zweiten Sauerstoffsättigungsgrad in dem Blut, der streng höher als der erste Sauerstoffsättigungsgrad in dem Blut ist;
• eine erste Herzfrequenz; und
• eine zweite Herzfrequenz, die streng niedriger als die erste Herzfrequenz ist,
- Bestimmen, anhand der erfassten Messungen, ob eine der folgenden ersten Bedingungen erfüllt ist:
• das Gewicht hat sich relativ zu dem Sollgewicht des Patienten um mehr als die erste Erhöhunggrenze des Gewichts erhöht;
• das Gewicht hat sich zwischen zwei Messungen, die über einen vorbestimmten Zeitraum von streng mehr als 2 Tagen durchgeführt wurden, um einen Wert erhöht, der streng über der ersten Erhöhunggrenze des Gewichts liegt;
• der Sauerstoffsättigungsgrad in dem Blut ist streng niedriger als der erste Sauerstoffsättigungsgrad in dem Blut;
• die Herzfrequenz ist streng höher als die erste Herzfrequenz;
- Bestimmen, dass eine beginnende Herzdekompensation wahrscheinlich ist, wenn mindestens eine der ersten Bedingungen erfüllt ist;
- Bestimmen, anhand der erfassten Messungen, ob eine der folgenden zweiten Bedingungen erfüllt ist:
• das Gewicht hat sich zwischen zwei Messungen, die an 2 aufeinanderfolgenden Tagen durchgeführt wurden, um einen Wert erhöht, der streng höher als die zweite Erhöhunggrenze des Gewichts ist;
• der Sauerstoffsättigungsgrad in dem Blut ist streng niedriger als der zweite Sauerstoffsättigungsgrad in dem Blut;
• die Herzfrequenz ist streng höher als die zweite Herzfrequenz.
- Bestimmen, dass eine beginnende Herzdekompensation wahrscheinlich ist, wenn zwei der zweiten Bedingungen gleichzeitig erfüllt sind, während keine der ersten Bedingungen erfüllt ist.

2. Vorrichtung nach Anspruch 1, wobei das Sollgewicht einer ersten Gewichtsmessung oder einer ersten Messung nach einem Zurücksetzen der Vorrichtung auf null oder einer ersten Messung nach einem Zeitraum ohne Messung von mehr als 4 Tagen entspricht.

3. Vorrichtung nach Anspruch 1 oder 2, **dadurch gekennzeichnet, dass**
• die erste Erhöhunggrenze des Gewichts gleich 4 kg ist;
• die zweite Erhöhunggrenze des Gewichts gleich 2 kg ist;
• der vorbestimmte Zeitraum 4 Tage beträgt;
• der erste Sauerstoffsättigungsgrad in dem Blut gleich 90 % ist;
• der zweite Sauerstoffsättigungsgrad in dem Blut gleich 95 % ist;
• die erste Herzfrequenz gleich 110 Schläge/Min. ist;
• die zweite Herzfrequenz gleich 90 Schläge/Min. ist.

4. Vorrichtung nach Anspruch 1, 2 oder 3, **dadurch gekennzeichnet, dass** sie eine Uhr (25) umfasst, die mit einer Mensch-Maschine-Schnittstelle (27) verknüpft ist, um den Patienten daran zu erinnern, jeden Tag innerhalb eines vorbestimmten Zeitfensters seine Messungen durchzuführen.

5. Vorrichtung nach einem der vorstehenden Ansprüche,
**dadurch gekennzeichnet, dass** sie eine vierte Kommunikationsschnittstelle (23) umfasst, die angepasst ist, um die Messungen an einen Remote-Server (9) zu übertragen.

6. System für die Fernüberwachung eines an Herzinsuffizienz leidenden Patienten, umfassend:
• eine Fernüberwachungsvorrichtung (3) nach einem der Ansprüche 1 bis 5;
• eine Waage (5);
• ein Oximeter (7); wobei die Waage (5) und das Oximeter (7) mit der Fernüberwachungsvorrichtung (3) verbunden sind; und
• einen Remote-Server (9), der mit der Fernüberwachungsvorrichtung (3) verbunden ist und angepasst ist, um den Verlauf der Messungen des Patienten zu erfassen, speichern und darzustellen.

7. Computerprogrammprodukt, **dadurch gekennzeichnet, dass** es Programmcodeanweisungen für die Implementierung eines Verfahrens für die Fernüberwachung eines an Herzinsuffizienz leidenden Patienten durch die Vorrichtung nach einem der vorstehenden Ansprüche umfasst, wobei das Verfahren umfasst:
- Erhalten von Messungen des Patientengewichts über die erste Kommunikationsschnittstelle (11) mit einer Waage (5),
- Erhalten von Messungen der Herzfrequenz und des Sauerstoffsättigungsgrads in dem Blut des Patienten über die zweite Kommunikationsschnittstelle (13) mit einem Oximeter (7);
- Erhalten vorbestimmter Werte, umfassend:
• eine erste Erhöhunggrenze des Gewichts;
• eine zweite Erhöhunggrenze des Gewichts, die streng niedriger als die erste Erhöhunggrenze des Gewichts ist;
• einen ersten Sauerstoffsättigungsgrad in dem Blut;
• einen zweiten Sauerstoffsättigungsgrad in dem Blut, der streng höher als der erste Sauerstoffsättigungsgrad in dem Blut ist;
• eine erste Herzfrequenz; und
• eine zweite Herzfrequenz, die streng niedriger als die erste Herzfrequenz ist,
- Bestimmen anhand der für den Patienten erhaltenen Messungen von Gewicht, Herzfrequenz und Sauerstoffsättigung, ob eine der folgenden ersten Bedingungen erfüllt ist:
• das Gewicht hat sich relativ zu dem Sollgewicht des Patienten um einen Wert erhöht, der streng höher als die erste Erhöhunggrenze des Gewichts ist;
• das Gewicht hat sich zwischen zwei Messungen, die über einen vorbestimmten Zeitraum von streng mehr als 2 Tagen durchgeführt wurden, um einen Wert erhöht, der streng über der ersten Erhöhunggrenze des Gewichts liegt;
• der Sauerstoffsättigungsgrad in dem Blut ist streng niedriger als der erste Sauerstoffsättigungsgrad in dem Blut;
• die Herzfrequenz ist streng höher als die erste Herzfrequenz;
- Auslösen eines Alarms, der signalisiert, dass eine beginnende Herzdekompensation wahrscheinlich ist, wenn mindestens eine der ersten Bedingungen erfüllt ist;
- Bestimmen, anhand der erhaltenen Messungen, ob eine der folgenden zweiten Bedingungen erfüllt ist:
• das Gewicht hat sich zwischen zwei Messungen, die an 2 aufeinanderfolgenden Tagen durchgeführt wurden, um einen Wert erhöht, der streng höher als die zweite Erhöhunggrenze des Gewichts ist;
• der Sauerstoffsättigungsgrad in dem Blut ist streng niedriger als der zweite Sauerstoffsättigungsgrad in dem Blut;
• die Herzfrequenz ist streng höher als die zweite Herzfrequenz;
- Auslösen eines Alarms, der signalisiert, dass eine beginnende Herzdekompensation wahrscheinlich ist, wenn zwei der zweiten Bedingungen gleichzeitig erfüllt sind, während keine der ersten Bedingungen erfüllt ist.

8. Computerprogrammprodukt nach Anspruch 7, wobei das Sollgewicht einer ersten Gewichtsmessung, einer ersten Messung nach einem Zurücksetzen auf null oder einer ersten Messung nach einem Zeitraum ohne Messung von mehr als 4 Tagen entspricht.

9. Computerprogrammprodukt nach Anspruch 7 oder 8,
**dadurch gekennzeichnet, dass**
• die erste Erhöhunggrenze des Gewichts gleich 4 kg ist;
• die zweite Erhöhunggrenze des Gewichts gleich 2 kg ist;
• der vorbestimmte Zeitraum 4 Tage beträgt;
• der erste Sauerstoffsättigungsgrad in dem Blut gleich 90 % ist;
• die zweite Sauerstoffsättigungsrate in dem Blut gleich 95 % ist;
• die erste Herzfrequenz gleich 110 Schläge/Min. ist;
• die zweite Herzfrequenz gleich 90 Schläge/Min. ist.

## Claims

1. Device for remotely monitoring (3) a patient with chronic heart failure, said device comprising:
• a first communication interface (11) which is adapted for communicating with a weighing scale (5) in order to collect a measurement of the patient's weight;
• a second communication interface (13) which is adapted for communicating with an oxygen meter (7) in order to collect measurements of the patient's heart rate and blood oxygen saturation level;
• a third communication interface (15) which is adapted for transmitting a warning to the patient; and
• a computer (19) which is connected to the three interfaces (11, 13, 15) and comprises a memory (21) which is adapted for storing the collected measurements, the computer being adapted for triggering a warning when the collected measurements indicate a probable onset of cardiac decompensation in the patient;
the computer being configured to:
- obtain a patient's nominal weight and predetermined values comprising:
• a first weight increase limit;
• a second weight increase limit which is strictly less than the first weight increase limit;
• a first blood oxygen saturation level;
• a second blood oxygen saturation level which is strictly higher than the first blood oxygen saturation level;
• a first heart rate; and
• a second heart rate which is strictly lower than the first heart rate,
- determine, from the collected measurements, whether any of the following first conditions is met:
• the weight has increased by more than the first weight increase limit relative to the patient's nominal weight;
• the weight has increased, between two measurements taken over a predetermined period which is strictly more than 2 days, by a value which is strictly more than the first weight increase limit;
• the blood oxygen saturation level is strictly lower than the first blood oxygen saturation level;
• the heart rate is strictly higher than the first heart rate;
- determine that an onset of cardiac decompensation is probable when at least one of the first conditions is met;
- determine, from the collected measurements, whether any of the following second conditions is met:
• the weight has increased, between two measurements taken on 2 consecutive days, by a value which is strictly more than the second weight increase limit;
• the blood oxygen saturation level is strictly lower than the second blood oxygen saturation level;
• the heart rate is strictly higher than the second heart rate;
- determine that an onset of cardiac decompensation is probable when two of the second conditions are met simultaneously, while none of the first conditions is met.

2. Device according to claim 1, wherein the nominal weight corresponds to a first weight measurement or a first measurement after resetting the device or a first measurement after a period of more than 4 days without measurements.

3. Device according to claim 1 or claim 2, **characterized in that**
• the first weight increase limit is equal to 4 kg;
• the second weight increase limit is equal to 2 kg;
• the predetermined period is 4 days;
• the first blood oxygen saturation level is 90%;
• the second blood oxygen saturation level is 95%;
• the first heart rate is equal to 110 beats/min;
• the second heart rate is equal to 90 beats/min.

4. Device according to claim 1, claim 2 or claim 3, **characterized in that** it comprises a clock (25) which is associated with a human-machine interface (27) for reminding the patient to take their measurements each day within a predetermined time band.

5. Device according to any of the preceding claims, **characterized in that** it comprises a fourth communication interface (23) which is adapted for transmitting the measurements to a remote server (9).

6. System for remotely monitoring a patient with heart failure, comprising:
• a remote monitoring device (3) according to any of claims 1 to 5;
• a weighing scale (5);
• an oxygen meter (7); the weighing scale (5) and the oxygen meter (7) being connected to the remote monitoring device (3); and
• a remote server (9) which is connected to the remote monitoring device (3) and is adapted for collecting, storing and displaying the patient's measurement history.

7. Computer program product, **characterized in that** it comprises program code instructions for carrying out, by means of the device of any of the preceding claims, a method for remotely monitoring a patient with heart failure, the method comprising:
- obtaining, via the first communication interface (11) with a weighing scale (5), measurements of the patient's weight,
- obtaining, via the second communication interface (13) with an oxygen meter (7), measurements of the patient's heart rate and blood oxygen saturation level;
- obtaining predetermined values comprising:
• a first weight increase limit;
• a second weight increase limit which is strictly less than the first weight increase limit;
• a first blood oxygen saturation level;
• a second blood oxygen saturation level which is strictly higher than the first blood oxygen saturation level;
• a first heart rate; and
• a second heart rate which is strictly lower than the first heart rate,
- determining, from the weight, heart rate and oxygen saturation level measurements obtained for the patient, whether any of the following first conditions is met:
• the weight has increased by a value which is strictly more than the first weight increase limit relative to the patient's nominal weight;
• the weight has increased, between two measurements taken over a predetermined period which is strictly more than 2 days, by a value which is strictly more than the first weight increase limit;
• the blood oxygen saturation level is strictly lower than the first blood oxygen saturation level;
• the heart rate is strictly higher than the first heart rate;
- triggering a warning which indicates that an onset of cardiac decompensation is probable when at least one of the first conditions is met;
- determining, from the obtained measurements, whether any of the following second conditions is met:
• the weight has increased, between two measurements taken on 2 consecutive days, by a value which is strictly more than the second weight increase limit;
• the blood oxygen saturation level is strictly lower than the second blood oxygen saturation level;
• the heart rate is strictly higher than the second heart rate;
- triggering a warning which indicates that an onset of cardiac decompensation is probable when two of the second conditions are met simultaneously, while none of the first conditions is met.

8. Computer program product according to claim 7, wherein the nominal weight corresponds to a first weight measurement, a first measurement after resetting or a first measurement after a period of more than 4 days without measurements.

9. Computer program product according to claim 7 or claim 8, **characterized in that**
• the first weight increase limit is equal to 4 kg;
• the second weight increase limit is equal to 2 kg;
• the predetermined period is 4 days;
• the first blood oxygen saturation level is 90%;
• the second blood oxygen saturation level is 95%;
• the first heart rate is equal to 110 beats/min;
• the second heart rate is equal to 90 beats/min.
